(19) Europäisches Patentamt / European Patent Office / Office européen des brevets

(11) **EP 3 616 532 B1**

(12) **EUROPEAN PATENT SPECIFICATION**

(45) Date of publication and mention
of the grant of the patent:
**18.06.2025 Bulletin 2025/25**

(21) Application number: **17908074.2**

(22) Date of filing: **22.11.2017**

(51) International Patent Classification (IPC):
*A23L 27/30* (2016.01)    *A61K 9/00* (2006.01)
*A23L 27/00* (2016.01)

(52) Cooperative Patent Classification (CPC):
**A23L 27/36; A23L 27/88;** A61K 9/0053

(86) International application number:
**PCT/JP2017/042098**

(87) International publication number:
**WO 2018/198411 (01.11.2018 Gazette 2018/44)**

(54) **SWEETENER COMPOSITION AND METHOD FOR IMPROVING TASTE OF STEVIA EXTRACT**

SÜSSSTOFFZUSAMMENSETZUNG UND VERFAHREN ZUR VERBESSERUNG DES
GESCHMACKS EINES STEVIA EXTRAKTS

COMPOSITION D'ÉDULCORANT ET PROCÉDÉ D'AMÉLIORATION DU GOÛT DE L'EXTRAIT DE
STÉVIA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2017 JP 2017089053**

(43) Date of publication of application:
**04.03.2020 Bulletin 2020/10**

(73) Proprietor: **San-Ei Gen F.F.I., INC.
Toyonaka-shi, Osaka 561-8588 (JP)**

(72) Inventors:
• **YOSHINAKA, Koji**
**Toyonaka-shi**
**Osaka 561-8588 (JP)**
• **KONDO, Akane**
**Toyonaka-shi**
**Osaka 561-8588 (JP)**

(74) Representative: **Vossius & Partner
Patentanwälte Rechtsanwälte mbB
Siebertstrasse 3
81675 München (DE)**

(56) References cited:
WO-A1-2009/063921    WO-A1-2016/112129
CN-B- 102 450 624    JP-A- 2010 535 022
US-A1- 2014 037 814

• DATABASE PubChem [O] 01112013;
REBAUDIOSIDE A, XP055528935, retrieved from
NCBI Database accession no. CID 71751991
• HEIMBACH J: "Determination of the GRAS
Status Of the Use Of Luo Han Fruit Concentrate
As a Flavor Modifier and Sweetener", 1 July 2009
(2009-07-01), pages 1 - 112, XP055640831,
Retrieved from the Internet <URL:https://1pdf.
net/download/
gras-notice-000301-luo-han-fruit-fdagov_58
ce3c30f6065d18370962d2>

## Description

Technical Field

The present invention relates to a sweetener composition, and a composition that is orally administered or used for the oral cavity comprising the same. Further, the present invention also relates to a method for improving the taste of stevia extract.

Background Art

Conventionally, sweeteners have been widely used to impart sweetness to foods, beverages, and medicines, as well as to adjust their taste. In particular, due to the recent rise in health concerns, non- or low-calorie high-intensity sweeteners, and high-intensity sweeteners with low caries-producing effects have been widely used. Examples of high-intensity sweeteners include synthetic sweeteners, such as aspartame, sucralose, acesulfame potassium, neotame, and advantame; and natural sweeteners, such as stevia extract, siraitia grosvenorii extract, and thaumatin. As a result of the recent trend towards natural products, natural sweeteners, such as stevia extract and siraitia grosvenorii extract, have become favored.

However, these natural sweeteners, particularly stevia extract, have a sweetness quality different from that of sugar, and have problems such as bitterness in their sweetness, a lingering sweetness (remaining sweetness) left in the mouth, a lack of richness in their sweetness, slow sweetness expression in the oral cavity, and insufficient sweetness quality. Rebaudioside A, which is a main sweetness component contained in stevia extract, has less lingering sweetness and rough taste, such as bitterness, compared with other sweetness components, such as stevioside, in the stevia extract; however, rebaudioside A still has the above problems. Moreover, mogroside V, which is a main sweetness component of siraitia grosvenorii extract, also has problems such as bitterness and unique flavor.

In order to solve these problems of stevia extract, conventionally, there have been proposals, for example, for methods using stevia extract in combination with a rutin derivative (PTL 1), royal jelly (PTL 2), quinic acid (PTL 3), cellooligosaccharide (PTL 4), vicenin-2 contained in fruit juices of citrus (PTL 5), or the like. Moreover, PTL 6 proposes a method for improving the taste of stevia extract by mixing thaumatin at a specific ratio relative to the stevia extract. Further, PTL 7 indicates that a composition having an excellent sweetness quality is obtained by mixing rebaudioside A, which is a main sweetness component of stevia extract, with mogroside V, which is a main sweetness component of siraitia grosvenorii extract, at a weight ratio of 95:5 to 60:40. In addition, PTL 8 discloses a composition comprising mogroside V and a rebaudioside component, such as rebaudioside A, at a weight ratio ranging from 1:1 to 6:1.

Citation List

Patent Literature

PTL 1: JPH10-146165A
PTL 2: JP2000-287642A
PTL 3: JP2001-321115A
PTL 4: JP2002-223721A
PTL 5: JP2006-238828A
PTL 6: JP2011-24445A
PTL 7: WO2009/063921
PTL 8: JP2016-41073A

Summary of Invention

Technical Problem

The present invention is defined by the claims.

Solution to Problem

The present inventors conducted extensive research to achieve the above objects, and consequently found that rebaudioside A, which is a main sweetness component of stevia extract, was mixed with a slight amount of mogroside V, which is a main sweetness component of siraitia grosvenorii extract, so that the proportion of rebaudioside A to mogroside V was 96:4 to 98:2 (mass ratio), whereby the problems (bitterness, lingering sweetness, lack of richness, and slow sweetness expression) of the sweetness quality of stevia extract and its main sweetness component, rebaudioside A, were reduced or solved, and a composition having excellent taste closer to that of sugar was obtained. In particular, it was confirmed that the combined use of rebaudioside A and mogroside V with the above proportion accelerated the expression of sweetness in the oral cavity and enhanced richness, thereby synergistically solving the problem of stevia extract, i.e., insufficient sweetness quality. These results suggest that the use of this composition as a sweetener can impart excellent sweetness to various products, such as foods and beverages, drugs, and quasi-drugs, which are orally administered or used for the oral cavity.

The present invention has been completed based on these findings, and has the following embodiments.

(I) Sweetener Composition, and Composition Orally Administered or Used for the Oral Cavity Comprising the Same

A sweetener composition comprising a stevia extract comprising 90 mass% or more of rebaudioside A, and mogroside V, wherein rebaudioside A and mogroside V are mixed in the sweetener composition at a mass ratio of rebaudioside A to mogroside V of 96:4 to 98:2.

In the sweetener composition the total amount of stevioside and rebaudioside C contained in the stevia extract can be 0.2 mass% or less.

A composition orally administered or used for an oral cavity, comprising the sweetener composition according to any one of appended claims 1 or 2, wherein the mass ratio of rebaudioside A to mogroside V in the oral composition is 96:4 to 98:2.

The composition orally administered or used for an oral cavity can be a food or beverage composition.

The composition orally administered or used for an oral cavity can comprise rebaudioside A and mogroside V in a total amount of 10 ppm to 5000 ppm.

(II) Method for Imparting Sweetness to Composition Orally Administered or Used for the Oral Cavity

A method for imparting sweetness to a composition orally administered or used for an oral cavity, wherein a stevia extract comprising 95 mass% or more of rebaudioside A, and mogroside V are mixed in the composition orally administered or used for an oral cavity at a mass ratio of rebaudioside A to mogroside V of 96:4 to 98:2.

In the sweetness-imparting method according to appended claim 6 the total amount of stevioside and rebaudioside C contained in the stevia extract can be 0.2 mass% or less.

The sweetness-imparting method according to appended claim 6 or 7 , wherein the composition orally administered or used for an oral cavity can comprise rebaudioside A and mogroside V in a total amount of 10 ppm to 5000 ppm.

(III) Method for Improving Taste of Stevia Extract

A method for improving the taste of a stevia extract comprising 90 mass% or more of rebaudioside A, comprising the step of:
mixing the stevia extract with mogroside V at a mass ratio of rebaudioside A to mogroside V within a range of 96:4 to 98:2.

In the taste-improving method according to appended claim 9 the total amount of stevioside and rebaudioside C contained in the stevia extract can be 0.2 mass% or less.

In the taste-improving method the taste to be improved can be at least one selected from the group consisting of the bitterness, lingering sweetness, richness, and quickness of sweetness expression of the stevia extract, preferably all of them.

Advantageous Effects of Invention

The method for improving the taste of stevia extract of the present invention can improve problems in the sweetness quality of stevia extract (e.g., bitterness, lingering sweetness, a lack of richness, and slow sweetness expression), and can provide a sweetener composition having an excellent sweetness quality closer to that of sugar using stevia extract, which is a natural sweetener, as a raw material. Moreover, the method for improving the taste of stevia extract of the present invention can improve the slow expression of sweetness in the oral cavity, which is a drawback of stevia extract, to accelerate the sweetness expression, thereby reducing or solving problems, such as insufficient sweetness quality and lingering sweetness (remaining aftertaste).

As a result of improvement in the problems (e.g., bitterness, lingering sweetness, lack of richness, and slow sweetness expression) of the sweetness quality of stevia extract and its sweetness component, stevioside A, the sweetener composition of the present invention has an excellent sweetness quality close to that of sugar. Accordingly, the sweetener composition of the present invention can impart an excellent sweetness quality to various compositions orally administered or used for the oral cavity.

Description of Embodiments

(I) Sweetener Composition

The sweetener composition of the present invention (hereinafter simply referred to as "the present sweetener composition") is characterized by comprising rebaudioside A and mogroside V at a mass ratio of 96:4 to 98:2.

Rebaudioside A

Rebaudioside A is a steviol glycoside contained in stevia extract, and is known, as a main sweetness component of stevia extract, to have a degree of sweetness 300 to 450 times that of sugar. Rebaudioside A can be prepared by extraction from leafs, stems, or the like of Stevia rebaudiana Bertoni (abbreviated as "stevia" in the present invention), which is a plant belonging to the genus Stevia, family Compositae, with water or an organic solvent, followed by purification. The rebaudioside A targeted by the present invention includes enzyme-treated rebaudioside A obtained by transferring sugar, such as glucose or fructose, to rebaudioside A using $\alpha$-glucosyltransferase or the like.

In the present sweetener composition, rebaudioside A may be used in a purified state, but is not limited thereto. To the extent that the working effect of rebaudioside A in the present sweetener composition is not prevented, rebaudioside A can also be used as a mixture with other steviol glycosides (stevioside, rebaudioside B, rebaudioside C, rebaudioside D, rebaudioside, dulcoside A, rubusoside, steviolbioside, etc.). Preferable examples of such mixtures include stevia extract. The stevia extract includes enzyme-treated stevia obtained by transferring sugar, such as glucose or fructose, to the stevia extract mentioned above using $\alpha$-glucosyltransferase or the like. When such a mixture is used, the content of rebaudioside A in the mixture is preferably 90 mass% or more, and more preferably 95 mass% or more, of the total amount. This is because if the content of components other than rebaudioside A in the mixture increases to more than 5 mass%, the influence of these components on the taste of the present sweetener composition cannot be ignored. Preferable examples of the mixture include, but are not limited to, stevia extract containing rebaudioside A in an amount of 95 mass% or more of the total amount, and other steviol glycosides, as other components, in a total amount of 1 mass% or less, and more preferably stevioside and rebaudioside C in a total amount of 0.2 mass% or less.

The rebaudioside A can be prepared by subjecting leaves, stems, or the like of Stevia Rebaudiana Bertoni as raw materials to extraction and purification. Simply, commercially available products can also be used. Examples of such products include Rebaudio J-100 (produced by Morita Kagaku Kogyo Co., Ltd.) and the like. This product is a rebaudioside A-containing product (stevia extract) comprising 95 mass% or more of rebaudioside A.

Mogroside V

Mogroside V is a triterpene-based glucoside contained in siraitia grosvenorii extract, and is known, as a main sweetness component of siraitia grosvenorii extract, to have a degree of sweetness about 300 times that of sugar. Mogroside V can be prepared by extraction with water from raw fruits of Rakanka (scientific name: Siraitia grosvenorii), which is a plant belonging to the genus Siraitia, family Cucurbitaceae, followed by purification.

In the present sweetener composition, mogroside V may be used in a purified state, as with rebaudioside A, but is not limited thereto. To the extent that the working effect of mogroside V in the present sweetener composition is not prevented, mogroside V can be used as a mixture with other triterpene-based glycosides (mogrol, mogroside $IE_1$, mogroside $IA_1$, mogroside IIE, mogroside III, mogroside IVa, mogroside IVE, simenoside, 11-oxo-mogroside, and $5\alpha,6\alpha$-epoxymogroside). Preferable examples of such mixtures include siraitia grosvenorii extract. When such a mixture is used, the amount of mogroside V in the mixture is preferably 30 mass% or more, and more preferably 50 mass% or more, of the total amount. This is because if the contents of components other than mogroside V in the mixture increases, the influence of these components on the taste of the present sweetener composition cannot be ignored.

The mogroside V can be prepared by subjecting fruits of Siraitia grosvenorii as raw materials to extraction and purification, as described above. Simply, commercially available products can also be used. Examples of such products include, but are not limited to, high-purity siraitia grosvenorii extract containing a high purity of mogroside V and having a degree of sweetness about 300 times that of sugar (produced by Saraya Co., Ltd.).

Present Sweetener Composition

The present sweetener composition is characterized by comprising rebaudioside A and mogroside V at a mass ratio 96:4 to 98:2, by mass ratio. As shown in Experimental Examples, provided later, when the amount of mogroside V is less than 1 part by mass and more than 5 parts by mass based on 100 parts by mass of the total amount of rebaudioside A and mogroside V, the effect of improving the sweetness quality of rebaudioside A cannot sufficiently be obtained. Moreover, because mogroside V is expensive, use of more than 5 parts by mass of mogroside V causes a problem of increasing product costs.

The present sweetener composition can be produced by mixing rebaudioside A and mogroside V at the ratio described above. The present sweetener composition can also be produced by using the above-mentioned stevia extract comprising 95 mass% or more of rebaudioside A, and mixing it with mogroside V so that the mass ratio of rebaudioside A to mogroside V is, as described above, 96:4 to 98:2. As the mogroside V used herein, siraitia grosvenorii extract comprising mogroside V as a main sweetness component can also be used, to the extent that the effects of the present invention are not impaired. Thus, the defects (bitterness, lingering sweetness, lack of richness, and slow sweetness expression) of the sweetness quality of stevia extract can be improved, and a sweetener composition having an excellent sweetness quality closer to that of sugar can be obtained.

The present sweetener composition is used to impart sweetness to, for example, a target composition that is orally administered or used for the oral cavity, described later. There is no limitation to the form thereof, and the present sweetener composition may take any form, including a solid form, such as a powder, a granule, a tablet, or a capsule; or a semi-solid or liquid form, such as a syrup, an emulsion, a liquid, or a gel. Moreover, the present sweetener composition may take a single-agent or two-agent form.

When the present sweetener composition is produced by mixing rebaudioside A and mogroside V at the ratio described above (or by mixing stevia extract with mogroside V so that the proportion of rebaudioside A to mogroside V satisfies the above-described proportion), pharmaceutically acceptable carriers or carriers that can be added to foods and beverages can be suitably added, depending on the form thereof. Examples of such carriers include, within a range that does not affect the taste of the present sweetener composition, oligosaccharides, such as isomalt-oligosaccharide, galacto-oligosaccharide, and fructo-oligosaccharide; polysaccharides, such as dextrin, cellulose, gum arabic, and starch (e.g., corn starch); water; and the like. Moreover, to the extent that the taste of the present sweetener composition is not affected, mixing of the following components is not excluded: saccharides, such as lactose, glucose, fructose, sugar, and high-fructose corn syrup; sugar alcohols, such as sorbitol, erythritol, lactitol, maltitol, mannitol, xylitol, and reduced-palatinose; synthetic sweeteners, such as aspartame, sucralose, acesulfam potassium, saccharin, and salts thereof (e.g., sodium saccharin and calcium saccharin); natural sweeteners, such as licorice extract, amacha extract, brazzein, neohesperidine dihydrochalcone, serendipity berry extract (monellin), and tenryocha extract. Further, flavors, colorants, acidulants, preservatives, and other components that are ordinarily used for foods, beverages, or medicines may be added within a range that does not affect the taste of the present sweetener composition.

The total ratio of rebaudioside A and mogroside V in the present sweetener composition can be suitably set within a range of 0.2 to 100 mass%, depending on the purpose of using the present sweetener composition, the use mode thereof, the presence or absence of other sweetness components, and the like. Since the degree of sweetness of the mixture (100 mass%) comprising rebaudioside A and mogroside V at the ratio described above is about 150 to 450 times that of sugar, for example, when the total amount of sugar in an orally administered composition is replaced by the same amount of the present sweetener composition, the total ratio of rebaudioside A and mogroside V contained in the present sweetener composition can be suitably adjusted within a range of about 0.2 to 0.7 mass%. Moreover, for example, when the present sweetener composition is directly used as a tabletop sweetener, the total ratio of rebaudioside A and mogroside V in the present sweetener composition can be suitably adjusted within a range of about 0.2 to 7 mass%, although it is not limited thereto. In this case, the present sweetener composition, in the same amount as sugar, has a degree of sweetness 1 to 10 times that of sugar; thus, sweetness similar to that of sugar can be obtained when the present sweetener composition is used in the same amount or up to 1/10 the amount of sugar.

(II) Composition Orally Administered or Used for the Oral Cavity

The composition orally administered or used for the oral cavity of the present invention (hereinafter abbreviated as "the present oral composition") is a composition having sweetness and comprising rebaudioside A and mogroside V at a mass ratio of 96:4 to 98:2. To this limitation, the present oral composition may contain mogroside V and stevia extract comprising 95 mass% or more of rebaudioside A. The proportion of rebaudioside A to mogroside V is 96:4 to 98:2 (mass ratio). The present oral composition can be easily prepared by adding and mixing the present sweetener composition described above to a target composition orally administered or used for the oral cavity described later.

The present oral composition targeted by the present invention is a composition that needs sweetness. Examples of such compositions include foods and beverages; orally administered medicines; medicines for the oral cavity; and oral care products, such as dentifrices and mouthwashes (including drugs or quasi drugs). Among these, foods and beverages are preferable.

Specific examples of foods and beverages include carbonated beverages, fruit-juice beverages, coffee beverages, black tea beverages, lactic acid beverages, lactic acid bacterium beverages, soft drinks, milk beverages, alcoholic beverages, and like beverages; okaki (small rice crackers), senbei (rice crackers), okoshi (dry cake made of rice or millet and starch syrup), manju (steamed azuki bean jam-filled bun), and various other Japanese sweets; cookies, biscuits, crackers, pies, sponge cakes, kasutera (Castella cakes), doughnuts, waffles, butter cream, custard cream, cream puffs, chocolate, chocolate confectioneries, caramel candies, chewing gum, jelly, pancakes, breads, and other Western-style confectioneries; yogurt, pudding, Bavarian cream, and like milk products; candies; chewing gum, bubble gum, and like gums; potato chips and various other dry snacks; ice cream, popsicles, sherbet, and other ice confectioneries; flour paste, peanut paste, fruit paste, and other various pastes; pickles; miso, powdered miso, soy sauce, powdered soy sauce, moromi, fish sauce, sauce, ketchup, mayonnaise, solid bouillon, sauces used for yakiniku, base for stew, base for soup, base for lightly pickled foods, and other seasonings.

Specific examples of orally administered medicines include troches, ampuled liquid medicines, granules, pulvis (powders), tablets, and capsules. Specific examples of medicines for the oral cavity include sprays, ointments, creams, pastes, and patches. Specific examples of oral care products include liquid dentifrices, toothpastes, mouthwashes, and breath fresheners.

The present oral composition comprises rebaudioside A and mogroside V so that the proportion thereof falls within the above-mentioned range, and so that the final total concentration thereof in the present oral composition is 10 ppm to 5000 ppm. The final total concentration is preferably 25 ppm to 3000 ppm, and more preferably 50 ppm to 2000 ppm, although it varies depending on the type of the target present oral composition. Rebaudioside A and mogroside V may be added to a composition orally administered or used for the oral cavity at any step of its production.

(III) Method for Imparting Sweetness to Composition Orally Administered or Used for the Oral Cavity

The method for imparting sweetness to a composition orally administered or used for the oral cavity of the present invention can be carried out by adding rebaudioside A and mogroside V to the compositions orally administered or used for the oral cavity targeted in (II) above at a mass ratio of 96:4 to 98:2.

As the rebaudioside A, stevia extract comprising 95 mass% or more of rebaudioside A can also be used. The proportion of rebaudioside A to mogroside V is 96:4 to 98:2 (mass ratio).

The amounts of rebaudioside A and mogroside V necessary to impart an excellent sweetness quality similar to that of sugar to these compositions orally administered or used for the oral cavity vary depending on, for example, the type of the target composition; however, the total amount of rebaudioside A and mogroside V is generally 10 ppm to 5000 ppm, preferably 25 ppm to 3000 ppm, and more preferably 50 ppm to 2000 ppm.

In the sweetness-imparting method of the present invention, rebaudioside A and mogroside V may be independently added to a target composition orally administered or used for the oral cavity so as to satisfy the above-mentioned proportions and amounts. Alternatively, the present sweetener composition described in (I) above may be added to a target composition orally administered or used for the oral cavity so as to satisfy the above-mentioned amounts.

(IV) Method for Improving Taste of Stevia Extract

The method for improving the taste of stevia extract of the present invention can be carried out by mixing stevia extract comprising rebaudioside A with mogroside V so that the proportion of rebaudioside A to mogroside V is within a range of 96:4 to 98:2 by mass ratio. The proportion of rebaudioside A to mogroside V is 96:4 to 98:2 (mass ratio).

The stevia extract comprising rebaudioside A targeted herein comprises 90 mass% or more, and preferably 95 mass% or more, of rebaudioside A. Examples include, but are not limited to, stevia extract containing, as other components, steviol glycosides other than rebaudioside A in a total amount of 1 mass% or less; and preferably stevia extract containing stevioside and rebaudioside C in a total amount of 0.2 mass% or less.

When mogroside V and rebaudioside A are mixed at the ratio described above, the problems of stevia extract, such as bitterness, lingering sweetness (remaining aftertaste), lack of richness, and slow sweetness expression in the oral cavity, can be improved, and the taste of stevia extract can be brought closer to that of sugar. Therefore, the taste of stevia extract to be improved by the present invention is at least one selected from the group consisting of bitterness, lingering sweetness, richness, and sweetness expression speed in the oral cavity. The qualities of taste are comprehensively improved, i.e., reduction or elimination of bitterness, reduction or elimination of lingering sweetness, enhancement of richness, and higher speed of sweetness expression in the oral cavity, whereby the taste of stevia extract comes closer to that of sugar, and an excellent sweetness quality is imparted.

In the present specification, the terms "contain" and "comprise" include the meanings of "substantially consist of" and "consist of."

Examples

The contents of the present invention are described in detail using the following Experimental Examples and Examples. However, the present invention is not limited thereto. In the following, experiments are performed at atmospheric pressure and ordinary temperature, unless otherwise specified. The term "%" indicates "mass%," and "part" indicates "part by mass," unless otherwise specified. The "*" symbol in the text indicates a product of San-Ei Gen F.F.I., Inc.

In the following Experimental Examples, stevia extract (Rebaudio J-100, produced by Morita Kagaku Kogyo Co., Ltd.) (dry powder) was used as a rebaudioside A-containing raw material. Rebaudio J-100 is a high-intensity sweetener having sweetness 360 to 460 times that of sugar and comprising 95.2% of rebaudioside A, 0.1% of rebaudioside C, and 0.1% of stevioside (see JP2011-115142A). In the following Experimental Examples, this product is referred to as "stevia extract."

Further, in the following Experimental Examples, siraitia grosvenorii extract (dry powder) was used as a mogroside V-containing raw material. The siraitia grosvenorii extract was prepared as a dry powder by extracting raw fruits (non-dried fruits) of siraitia grosvenorii with water, then collecting the water extraction liquid by filtration, and decolorizing and concentrating the water extraction liquid, followed by spray drying. In the following Experimental Examples, the thus-prepared siraitia grosvenorii extracts comprising 50% and 30% of mogroside V are referred to, respectively, as "siraitia grosvenorii extract 1" and "siraitia grosvenorii extract 2."

Experimental Example 1: Improvement in Taste of Sweetener Composition (1)

(1) Preparation of Test Sample

Using the stevia extract and siraitia grosvenorii extract 1 described above, rebaudioside A and mogroside V were mixed at the compounding ratios (mass ratios) shown in Table 1, thereby preparing three types of sweetener compositions (Example 1, and Comparative Examples 1 and 2). The compounding ratio of rebaudioside A and mogroside V shown in Table 1 means the compounding ratio (mass ratio) of each component when the total amount of rebaudioside A and mogroside V is defined as 100 parts (hereinafter the same in Tables 3 and 5). Each of these compositions was added to water to obtain a degree of sweetness (intensity of sweetness) similar to that of a 6% aqueous sugar solution, thereby preparing three types of aqueous solutions (test samples: Example 1-1, and Comparative Examples 1-1 and 2-1). Further, as a control sample, an aqueous solution was prepared by adding stevia extract (Control Example 1) to water to similarly obtain a degree of sweetness similar to that of a 6% aqueous sugar solution (Control Example 1-1).

Table 1

| Formulation of various sweetener compositions, and amount thereof added to water (concentration in test sample) part by mass/mass% | | | | |
|---|---|---|---|---|
| | Control Example 1 | Example 1 | Comparative Example 1 | Comparative Example 2 |
| Rebaudioside A (stevia extract) | 100 parts (100%) | 96.2 parts (93%) | 92.7 parts (87%) | 67.3 parts (52%) |
| Mogroside V (siraitia grosvenorii extract 1) | 0 parts (0%) | 3.8 parts (7%) | 7.3 parts (13%) | 32.7 parts (48%) |
| Total amount of rebaudioside A and mogroside V (total amount of sweetener composition) | 100 parts (100%) | 100 parts (100%) | 100 parts (100%) | 100 parts (100%) |
| Amount of sweetener composition added to water (corresponding to the amount of 6% sugar) | 0.026% | 0.023% | 0.024% | 0.025% |

(2) Evaluation of Taste

Six trained panelists engaged in the sensory evaluation of the taste of sweeteners were asked to evaluate the sweetness quality of the test samples (Example 1-1, and Comparative Examples 1-1 and 2-1) prepared above, from four viewpoints, i.e., rough taste (e.g., bitterness), lingering sweetness, richness, and quickness of sweetness expression felt in the oral cavity. Specifically, the sweetness quality of the control sample was set to 0 points, and the sweetness quality of a 6% aqueous sugar solution (positive control) was set to 5 points. As the sweetness quality of the test sample came closer to that of the 6% aqueous sugar solution from the control sample, points were added almost equally one by one. When the sweetness quality became the closest to that of the 6% aqueous sugar solution, the sweetness quality was given 5 points. From the average values of the six panelists, comprehensive evaluation was conducted according to the following criteria.

Evaluation Criteria

+++: The average value was 4 or more.

++: The average value was 3 or more and less than 4.

+: The average value was 2 or more and less than 3.

±: The average value was 1 or more and less than 2.

-: The average value was less than 1.

Table 2 shows the results.

Table 2

| Control sample/ test sample | Comprehensive evaluation | Specific evaluation |
|---|---|---|
| Control Example 1-1 | 0 points | Rough taste, such as bitterness, was felt. Lingering sweetness was felt. Richness was barely felt. Sweetness expression in the oral cavity was slightly slow. |
| Example 1-1 | +++ | There was less rough taste, such as bitterness. Lingering sweetness was barely felt. Richness was imparted, and rough taste was hardly felt. Quickness of sweetness expression in the oral cavity was close to that of sugar. |
| Comparative Example 1-1 | + | There was less rough taste, such as bittemess. Lingering sweetness was slightly felt. Richness was imparted, and rough taste was hardly felt. Quickness of sweetness expression in the oral cavity was slightly close to that of sugar. |
| Comparative Example 2-1 | ± | Rough taste, such as bitterness, was slightly felt. Lingering sweetness was felt. Richness was imparted, and rough taste was hardly felt. Quickness of sweetness expression in the oral cavity slightly came close to that of sugar. |

These results confirmed that the sweetener composition comprising rebaudioside A and mogroside V at a mass ratio of 96.2:3.8 (Example 1) had less lingering sweetness and had quickness of sweetness expression in the oral cavity closer to that of sugar, compared with the sweetener composition comprising rebaudioside A and mogroside V at mass ratio of 92.7:7.3 (Comparative Example 1). Further, as is clear from the results of Comparative Examples 1 and 2, when the total amount of rebaudioside A and mogroside V was set to 100 parts, as the ratio of mogroside V increased to 5 parts or more (conversely, as the ratio of rebaudioside A decreased to 95 parts or less), bitterness and lingering sweetness became stronger, and the sweetness quality tended to become further from that of sugar, in terms of quickness of sweetness expression in the oral cavity. This revealed that a sweetener composition having a sweetness quality close to that of sugar was obtained by adjusting the proportion of rebaudioside A to mogroside V within a range of 95:5 to 99:1 (mass ratio).

Experimental Example 2: Improvement in Taste of Sweetener Composition (2)

(1) Preparation of Test Sample

Using the stevia extract and siraitia grosvenorii extract 1 or siraitia grosvenorii extract 2 described above, rebaudioside A and mogroside V were mixed at the compounding ratios (mass ratios) shown in Table 3, thereby preparing two types of sweetener compositions (Examples 2 and 3). Each of these compositions was added to water to obtain a degree of sweetness (intensity of sweetness) similar to that of a 6% aqueous sugar solution, thereby preparing two types of aqueous solutions (test samples: Examples 2-1 and 3-1). Further, as a control sample, an aqueous solution of stevia extract (Control Example 1) adjusted to obtain a degree of sweetness similar to that of a 6% aqueous sugar solution (Control Example 1-1) was used, as in Experimental Example 1.

Table 3

| Formulation of various sweetener compositions, and amount thereof added to water (concentration in test sample) part by mass/mass% | | | |
|---|---|---|---|
| | Control Example 1 | Example 2 | Example 3 |
| Rebaudioside A (stevia extract) | 100 parts (100%) | 97.3 parts (95%) | 97.3 parts (91.8%) |
| Mogroside V (siraitia grosvenorii extract 1) | 0 parts (0%) | 2.7 parts (5%) | - (-) |
| Mogroside V (siraitia grosvenorii extract 2) | 0 parts (0%) | - (-) | 2.7 parts (8.2%) |
| Total amount of rebaudioside A and mogroside V (total amount of sweetener composition) | 100 parts (100%) | 100 parts (100%) | 100 parts (100%) |
| Amount of sweetener composition added to water (corresponding to the amount of 6% sugar) | 0.026% | 0.026% | 0.00269% |

(2) Evaluation of Taste

Three well-trained panelists were asked to evaluate the sweetness quality of the test samples (Examples 2-1 and 3-1) prepared above, in the same manner as in Experimental Example 1. The sweetness quality of the control sample 1-1 was set to 0 points, and the sweetness quality of a 6% aqueous sugar solution (positive control) was set to 5 points. Table 4 shows the results.

Table 4

| | Comprehensive evaluation | Specific evaluation |
|---|---|---|
| Control Example 1-1 | 0 points | Rough taste, such as bitterness, was felt. Lingering sweetness was felt. Richness was barely felt. Sweetness expression in the oral cavity was slightly slow. |
| Example 2-1 | +++ | There was less rough taste, such as bitterness. Lingering sweetness was improved. Richness was felt, and there was mellow sweetness. Quickness of sweetness expression in the oral cavity was close to that of sugar. |
| Example 3-1 | ++ | There was less rough taste, such as bitterness. Lingering sweetness was improved. Richness was felt, and there was mellow sweetness. Quickness of sweetness expression in the oral cavity came close to that of sugar. |

As shown in Table 4, it was confirmed that when either siraitia grosvenorii extract 1 comprising 50% of mogroside V or siraitia grosvenorii extract 2 comprising 30% of mogroside V was used as a mogroside V-containing raw material, a composition having less rough taste (e.g., bitterness) and lingering sweetness, having richness, and having a sweetness quality close to that of sugar was obtained by adjusting the proportion of rebaudioside A to mogroside V in the sweetener composition to 97.3:2.7 (mass ratio). This revealed that, among the components contained in the siraitia grosvenorii extract, mogroside V contributed to the improvement in the sweetness quality of rebaudioside A, particularly the improvement in the sweetness quality of stevia extract comprising 95% or more of rebaudioside A; and that a sweetener composition having a sweetness quality close to that of sugar was obtained by adjusting the proportion of rebaudioside A to mogroside V contained in the stevia extract within a range of 95:5 to 99:1 (mass ratio).

Experimental Example 3: Improvement in Taste of Sweetener Composition (3)

(1) Preparation of Test Sample

Using the stevia extract and siraitia grosvenorii extract 1 described above, rebaudioside A and mogroside V were mixed at the compounding ratios (mass ratios) shown in Table 5, thereby preparing three types of sweetener compositions (Example 2, and Comparative Examples 3 and 4). As shown in Table 6, these compositions were each added to an aqueous solution (acid aqueous solution) containing 0.1% citric acid and 0.03% trisodium citrate to obtain a degree of sweetness (intensity of sweetness) similar to that of an acid aqueous solution containing sugar at a concentration of 8% (containing 0.1% citric acid and 0.03% trisodium citrate), thereby preparing three types of acid sugar solutions (pH 3.2) (Example 2-2, and Comparative Examples 3-1 and 4-1). Further, as a control sample, an acid sugar solution was prepared by adding stevia extract (Control Example 1) to the above acid aqueous solution so as to obtain a degree of sweetness similar to that of an acid aqueous solution containing sugar at a concentration of 8% (Control Example 1-2).

Table 5

| Formulation of various sweetener compositions part by mass/mass% | | | | |
|---|---|---|---|---|
| | Control Example 1 | Example 2 | Comparative Example 3 | Comparative Example 4 |
| Rebaudioside A (stevia extract) | 100 parts (100%) | 97.3 parts (95%) | 94.5 parts (90%) | 81.6 parts (70%) |
| Mogroside V (siraitia grosvenorii extract 1) | 0 parts (0%) | 2.7 parts (5%) | 5.5 parts (10%) | 18.4 parts (30%) |
| Total amount of rebaudioside A and mogroside (total amount of sweetener composition) | 100 parts (100%) | 100 parts (100%) | 100 parts (100%) | 100 parts (100%) |

Table 6

| Amount of sweetener composition added to acid aqueous solution (concentration in test sample) mass% | | | | |
|---|---|---|---|---|
| Acid sugar solution (test sample) | Control Example 1-2 | Example 2-2 | Comparative Example 3-1 | Comparative Example 4-1 |
| Sweetener composition (Control Example 1, Example 2, Comparative Example 3, Comparative Example 4) | 0.0489% | 0.0489% | 0.0455% | 0.0500% |
| Citric acid | 0.1% | 0.1% | 0.1% | 0.1% |
| Trisodium citrate | 0.03% | 0.03% | 0.03% | 0.03% |
| Water | Balance | Balance | Balance | Balance |
| **Total** | 100% | 100% | 100% | 100% |

(2) Evaluation of Taste

Five well-trained panelists were asked to evaluate the sweetness quality of the test samples (Example 2-2, and Comparative Examples 3-1 and 4-1) prepared above. Specifically, the sweetness quality of the control sample (Control Example 1-2) was set to 0 points, and the sweetness quality of an 8% aqueous sugar solution (positive control) was set to 5 points. From two viewpoints, i.e., rough taste (e.g., bitterness) and lingering sweetness, as the sweetness quality of the test sample came closer to that of the 8% aqueous sugar solution from the control sample 1-2, points were added one by one. When the sweetness quality became the closest to that of the 8% aqueous sugar solution, the sweetness quality was given 5 points. From the average scores of the five panelists, comprehensive evaluation was conducted according to the following criteria.

Evaluation Criteria

+++: The average value was 4 or more.
++: The average value was 3 or more and less than 4.
+: The average value was 2 or more and less than 3.
±: The average value was 1 or more and less than 2.
-: The average value was less than 1.

Table 7 shows the results.

Table 7

| | Comprehensive evaluation | Specific evaluation |
|---|---|---|
| Control Example 1-2 | 0 points | There was rough taste, such as bitterness. Lingering sweetness was felt. |
| Example 2-2 | +++ | Rough taste, such as bitterness, was suppressed. Lingering sweetness was a little, and there was clean taste. |
| Comparative Example 3-1 | + | There was, but a little, rough taste, such as bitterness. There was, but a little, lingering sweetness. |
| Comparative Example 4-1 | ± | There was slight rough taste, such as bitterness. There was lingering sweetness. |

These results confirmed that the sweetener composition comprising rebaudioside A and mogroside V at a mass ratio of 97.3:2.7 (Example 2) had less bitterness and lingering sweetness, and the sweetness quality was closer to that of sugar even under acid conditions (pH 3.2) of an acid sugar solution, compared with the sweetener composition comprising rebaudioside A and mogroside V at a mass ratio of 94.5:5.5 (Comparative Example 3). Further, as is clear from the results of Comparative Examples 3-1 and 4-1, when the total amount of rebaudioside A and mogroside V was set to 100 parts, as the ratio of mogroside V increased to 5 parts or more (conversely, as the ratio of rebaudioside A decreased to 95 parts or less), bitterness and lingering sweetness occurred, and the sweetness quality tended to become further from that of sugar. This revealed that even in the case of use under acidic conditions, a sweetener composition having a sweetness quality close to that of sugar was obtained by adjusting the proportion of rebaudioside A to mogroside V contained in the stevia extract within a range of 95:5 to 99:1 (mass ratio).

Example 5: Application to Coffee Beverages

The sweetener compositions of Example 2 and Comparative Example 4, each in an amount equivalent to 3% sugar, were added to a beverage obtained by dissolving commercially available instant coffee "Blendy" (produced by Ajinomoto AGF, Inc.) in hot water, thereby preparing warm coffee beverages (temperature: 70°C) with sweetness. Three well-trained panelists were asked to taste these beverages and compare their taste.
Table 8 shows the formulations of the coffee beverages and the sensory evaluation results.

Table 8

|  |  | Example 5 | Comparative Example 5 |
|---|---|---|---|
|  |  |  | mass% |
| Sweetener composition (Example 2) (A:V = 97.3:2.7)[Note 1] | | 0.033 | - |
| Sweetener composition (Comparative Example 4) (A:V = 81.6:18.4)[Note 1] | | - | 0.033 |
| Instant coffee (powder) | | 1.25 | 1.25 |
| How water (90°C) | | Balance | Balance |
| Total | | 100 | 100 |
| Sensory evaluation | | Richness was increased, and mellow sweetness was imparted. Quickness of sweetness expression was close to that of sugar, and there was less lingering sweetness. | There was no richness. Sweetness expression was slow, and lingering sweetness was felt. Bittemess, sourness, and unpleasant taste that were not derived from coffee were felt. |
| **Note** 1: **Proportion of rebaudioside A (A) to mogroside** V (V) **(mass ratio)** | | | |

As shown in Table 8, it was confirmed that when mixed with a coffee beverage, the sweetener composition comprising rebaudioside A and mogroside V at a weight ratio of 97.3:2.7 (Example 2) imparted rich, mellow sweetness to the coffee beverage. It was also confirmed that because the quickness of sweetness expression came closer to that of sugar, a sweetness quality similar to that of sugar could be imparted (all Example 5). In contrast, when the sweetener composition comprising rebaudioside A and mogroside V at a weight ratio of 81.6:18.4 (Comparative Example 4) was mixed with a coffee beverage, there was no richness-enhancing effect. Further, there was lingering sweetness, and the sweetness quality was far away from that of sugar, in terms of slow sweetness expression (all Comparative Example 5). This reveals that the sweetener composition of the present invention is also useful as a sweetener that can improve the taste of coffee beverages.

Example 6: Application to Fruit-Juice Beverages

20% orange juice-containing beverages (Example 6 and Comparative Example 6) were prepared according to the formulations shown in Table 9. Five well-trained panelists were asked to taste these beverages and compare their taste. As the sensory evaluation, comparative evaluation was performed using, as a positive control (reference beverage), a 20% orange juice-containing beverage in which high-fructose corn syrup was mixed at a total concentration of 11%, in place of 0.033% of the sweetener composition mixed in Example 6 and Comparative Example 6, so as to obtain a similar degree of sweetness.
Table 9 also shows the sensory evaluation results.

Table 9

|  | Example 6 | Comparative Example 6 |
|---|---|---|
|  |  | mass% |
| 5-Fold concentrated orange mixed juice | 4 | 4 |
| High-fructose corn syrup | 3 | 3 |

(continued)

|  | | mass% |
| --- | --- | --- |
|  | Example 6 | Comparative Example 6 |
| Sweetener composition (Example 2) (A:V = 97.3:2.7)[Note 1] | 0.033 | - |
| Sweetener composition (Control Example 1) (A:V = 100:0)[Note 1] | - | 0.033 |
| Citric acid (anhydrous) | 0.15 | 0.15 |
| Trisodium citrate | 0.03 | 0.03 |
| Orange flavor No. 51237 (N)* | 0.1 | 0.1 |
| Water | Balance | Balance |
| Total | 100.0 | 100.0 |
| Comments | There was almost no lingering sweetness. Mellow sweetness similar to that of reference beverage was felt. There was richness in top, and bright orange flavor. | There was bitterness in the latter haft, and lingering sweetness. Richness was slightly felt, but there was orange flavor with bitterness. |
| Note 1: Proportion of rebaudioside A to mogroside V (mass ratio) | | |

Table 9 demonstrates that when Control Example 1, which contained only rebaudioside A but did not contain mogroside V, was used as a sweetener composition, there was bitterness in the latter half, and also lingering sweetness; and that when Example 2, which contained rebaudioside A and mogroside V at a weight ratio of 97.3:2.7, was used, there was almost no lingering sweetness, and a taste close to that of the reference beverage containing high-fructose corn syrup as sugar was recognized.

Example 7: Application to Sugar-Free Cookies

Sugar-free cookies (Example 7 and Comparative Example 7) were prepared according to the formulations shown in Table 10. Five well-trained panelists were asked to taste these cookies and compare their taste.

Table 10

|  | | | Kg |
| --- | --- | --- | --- |
|  | Example 7 | Comparative Example 7 | Control |
| 1. Wheat flour | 93.0 | 93.0 | 93.0 |
| 2. Ground almonds | 7.0 | 7.0 | 7.0 |
| 3. Polydextrose | 20.0 | 20.0 | - |
| 4. Maltitol | 15.0 | 15.0 | - |
| 5. Erythritol | 10.0 | 10.0 | - |
| 6. Caster sugar | - | - | 45.0 |
| 7. Stevia extract | - | 0.10 | - |

(continued)

| | Example 7 | Comparative Example 7 | Kg Control |
|---|---|---|---|
| 8. Sweetener composition (Example 2) | 0.11 | - | - |
| 9. Powdered skim milk | 2.0 | 2.0 | 2.0 |
| 10. Shortening | 20.0 | 20.0 | 20.0 |
| 11. Unsalted butter | 10.0 | 10.0 | 10.0 |
| 12. Whole egg | 20.0 | 20.0 | 20.0 |
| 13. Salt | 0.5 | 0.5 | 0.5 |
| 14. Swelling agent | 1.0 | 1.0 | 1.0 |
| 15. Flavor | 0.2 | 0.2 | 0.2 |
| 16. Water | 5.0 | 5.0 | 5.0 |
| Total before baking | 203.81 | 203.8 | 203.7 |

Production Method

1) Components 1 to 9, 13, and 14 were previously powder-mixed and sieved.
2) Components 10 and 11 were each weighed in the bowl of a universal mixer, and mixed with a beater.
3) The powder mixture of 1) was added to the resultant of 2) and mixed.
4) Components 12, 14, and 16 were added thereto and mixed.
5) The mixture (dough) was molded into a rod shape, and left in a refrigerator for 30 minutes.
6) After 30 minutes, the rod-shaped dough was taken from the refrigerator, sliced into 5 mm widths, and baked in an oven at 180°C for about 18 minutes, thereby producing cookies.

Evaluation

Example 7: There were less bitterness and lingering sweetness, and the taste was closer to that of the control cookie (sugar section), compared with Comparative Example 7.
Comparative Example 7: Bitterness and lingering sweetness were felt, compared with the control cookie (sugar section).

Example 8: Application to Vanilla Ice Cream (Ice Milk)

Vanilla ice cream (Example 8 and Comparative Example 8) was prepared according to the formulations shown in Table 11. Five well-trained panelists were asked to taste this ice cream and compare its taste.

Table 11

| | Example 8 | Comparative Example 8 | Kg Control |
|---|---|---|---|
| 1. Whole sweetened condensed milk | 5.0 | 5.0 | 5.0 |
| 2. Powdered skim milk | 6.0 | 6.0 | 6.0 |
| 3. Fresh cream | 9.0 | 9.0 | 9.0 |
| 4. Sugar | 2.0 | 2.0 | 5.5 |
| 5. Stevia extract | - | 0.014 | - |
| 6. Sweetener composition (Example 2) | 0.014 | - | - |

(continued)

|  | Example 8 | Comparative Example 8 | Kg Control |
|---|---|---|---|
| 7. Fructose | 3.0 | 3.0 | 3.0 |
| 8. Erythritol | 1.0 | 1.0 | 1.0 |
| 9. Dextrin | 1.0 | 1.0 | 1.0 |
| 10. Maltitol | 2.0 | 2.0 | 2.0 |
| 11. Indigestible dextrin | 4.0 | 4.0 | 4.0 |
| 12. Polydextrose | 4.0 | 4.0 | 4.0 |
| 13. Stabilizer | 0.3 | 0.3 | 0.3 |
| 14. Emulsifier | 0.2 | 0.2 | 0.2 |
| 15. Colorant | 0.05 | 0.05 | 0.05 |
| 16. Flavor | 0.1 | 0.1 | 0.1 |
| 17. Water | Balance | Balance | Balance |
| Total | 100.00 | 100.00 | 100.00 |

Production Method

1) While water and components 1 and 3 were stirred, a powder mixture of components 2 and 4 to 14 was added.
2) These components were dissolved by stirring under heating at 80°C for 10 minutes.
3) After weight correction, the resultant was homogenized with a homogenizer.
4) The resultant was allowed to stand at 5°C overnight for aging.
5) Components 15 and 16 were added thereto, and the resulting mixture was frozen to an overrun of 80%.
6) The resultant was placed in a cup, and cured by freezing at - 40°C.

In the production of frozen desserts, the term "overrun (OR)" refers to the increment of the volume of an ice cream mixture caused by freezing. The weight (A) of a cup of vanilla ice cream mixture before freezing is measured, the frozen vanilla ice cream is placed in the same cup to the top edge, the weight thereof (B) is measured, and OR is calculated from the following formula:

$$OR = (A - B/B) \times 100$$

Evaluation

Example 8: There were less bitterness and lingering sweetness, and the sweetness was closer to that of sugar, compared with Comparative Example 8. The sweetness was mellow, and strong milk flavor was felt.
Comparative Example 8: Bitterness and lingering sweetness were felt, compared with the control (sugar section).

Example 9: Application to Konnyaku Drink Jellies (Orange)

Drink jellies (Example 9 and Comparative Example 9) were prepared according to the formulations shown in Table 12. Five well-trained panelists were asked to taste these jellies and compare their taste.

Table 12

| | Example 9 | Comparative Example 9 | Control |
|---|---|---|---|
| | | | Kg |
| 1. Indigestible dextrin | 2.4 | 2.4 | 5.0 |
| 2. Erythritol | 3.0 | 3.0 | 6.0 |
| 3. High-fructose com syrup | - | - | 12.5 |
| 4. Stevia extract | - | 0.036 | - |
| 5. Sweetener composition (Example 2) | 0.036 | - | - |
| 6. Citric acid | 0.26 | 0.26 | 3.0 |
| 7. Trisodium citrate | 0.12 | 0.12 | 1.0 |
| 8. L-ascorbic acid | 0.05 | 0.05 | 1.0 |
| 9. Gelling agent | 0.6 | 0.6 | 2.0 |
| 10. Orange mixed juice | 2.0 | 2.0 | 4.0 |
| 11. Flavor | 0.3 | 0.3 | 4.0 |
| 12. Water | Balance | Balance | Balance |
| Total | 100.00 | 100.00 | 100.00 |

Production Method

1) While water and component 3 were stirred, a powder mixture of components 1, 2, 4, 5, 7, and 9 was added.
2) These components were dissolved by stirring under heating at 80°C for 10 minutes.
3) Components 8 and 10 to 12 were added thereto, and the pH was adjusted to 3.8.
4) The resultant was placed in a cup, and sterilized at 85°C for 30 minutes.
5) The resultant was cured by cooling.

Evaluation

Example 9: There were less bitterness and lingering sweetness, and the sweetness was closer to that of sugar, compared with Comparative Example 9. While bitterness was reduced, strong orange flavor was felt.
Comparative Example 9: Bitterness and lingering sweetness were felt, compared with the control (sugar section).

Example 10: Application to Non-Oil Dressings (Onion Flavor)

Dressings (Example 10 and Comparative Example 10) were prepared according to the formulations shown in Table 13. Five well-trained panelists were asked to taste these dressings and compare their taste.

Table 13

| | Example 10 | Comparative Example 10 |
|---|---|---|
| | | Kg |
| 1. Heavy soy sauce | 13.0 | 13.0 |
| 2. Stevia extract | - | 0.027 |
| 3. Sweetener composition (Example 2) | 0.027 | - |
| 4. Fermented vinegar | 10.0 | 10.0 |
| 5. Salt | 1.0 | 1.0 |

(continued)

|  | Kg | |
|---|---|---|
|  | Example 10 | Comparative Example 10 |
| 6. L-Sodium glutamate | 0.3 | 0.3 |
| 7. Seasoning (onion flavor) | 0.7 | 0.7 |
| 8. Seasoning (amino acid base) | 0.5 | 0.5 |
| 9. Seasoning (soup) | 0.2 | 0.2 |
| 10. Thickener | 0.1 | 0.1 |
| 11. Water | Balance | Balance |
| Total | 100.00 | 100.00 |

Production Method

1) Component 10 was added to water, and dissolved by stirring under heating at 80°C for 10 minutes.
2) All of the remaining components were added thereto, and the total amount was adjusted with water.
3) After heating to 93°C, the resultant was placed in a container.

Evaluation

Example 10: There were less bitterness and lingering sweetness, and strong onion flavor was felt.
Comparative Example 10: Bitterness and lingering sweetness were felt.

Example 11: Application to Gummy Candies (Orange)

Gummy candies (Example 11 and Comparative Example 11) were prepared according to the formulations shown in Table 14. Five well-trained panelists were asked to taste these gummy candies and compare their taste.

Table 14

|  | Kg | |
|---|---|---|
|  | Example 11 | Comparative Example 11 |
| 1. Maltitol | 53.0 | 53.0 |
| 2. Stevia extract | - | 0.22 |
| 3. Sweetener composition (Example 2) | 0.22 | - |
| 4. Reduced starch syrup | 42.0 | 42.0 |
| 5. Pectin | 0.3 | 0.3 |
| 6. Water | 30.0 | 30.0 |
| 7. 50% gelatin solution | 18.0 | 18.0 |
| 8. Citric acid | 1.4 | 1.4 |
| 9. Colorant | 0.05 | 0.05 |
| 10. Flavor | 0.4 | 0.4 |
| Weight before drying | 100.00 | 100.00 |

Production Method

1) Component 5 was dissolved in part of component 1. After dissolution by boiling, the remaining component 1 and components 4 and 6 were added and mixed.
2) The resulting mixture was simmered, and then cooled to 100°C. Then, component 7 was added and mixed.
3) Components 2, 3, and 8 to 10 were added thereto, mixed, and then molded.
4) After drying at room temperature, surface treatment was performed with a brightener.

Evaluation

Example 11: There were less bitterness and lingering sweetness, and strong orange flavor was felt.

**Claims**

1. A sweetener composition comprising a stevia extract comprising 90 mass% or more, preferably 95 mass% or more of rebaudioside A, and mogroside V, wherein rebaudioside A and mogroside V are mixed in the sweetener composition at a mass ratio of rebaudioside A to mogroside V of 96:4 to 98:2.

2. The sweetener composition according to claim 1, wherein the total amount of other steviol glycosides contained in the stevia extract is 1 mass% or less, and the total amount of stevioside and rebaudioside C contained in the stevia extract is 0.2 mass% or less.

3. A composition for oral administration or used for an oral cavity (oral composition),
comprising the sweetener composition according to claim 1 or 2, wherein the mass ratio of rebaudioside A to mogroside V in the oral composition is 96:4 to 98:2.

4. The oral composition according to claim 3, which is a food or beverage composition.

5. The oral composition according to claim 3 or 4, which comprises rebaudioside A and mogroside V in a total amount of 10 ppm to 5000 ppm.

6. A method for imparting sweetness to a composition for oral administration or used for an oral cavity (oral composition), wherein a stevia extract comprising 95 mass% or more of rebaudioside A, and mogroside V are mixed in the oral composition at a mass ratio of rebaudioside A to mogroside V of 96:4 to 98:2.

7. The sweetness-imparting method according to claim 6, wherein the total amount of other steviol glycosides contained in the stevia extract is 1 mass% or less, and the total amount of stevioside and rebaudioside C contained in the stevia extract is 0.2 mass% or less.

8. The sweetness-imparting method according to claim 6 or 7, wherein the oral composition comprises rebaudioside A and mogroside V in a total amount of 10 ppm to 5000 ppm.

9. A method for improving the taste of a stevia extract comprising 90 mass% or more, preferably 95 mass% or more of rebaudioside A, comprising the step of:
mixing the stevia extract with mogroside V at a mass ratio of rebaudioside A to mogroside V within a range of 96:4 to 98:2.

10. The taste-improving method according to claim 9, wherein the total amount of other steviol glycosides contained in the stevia extract is 1 mass% or less, and the total amount of stevioside and rebaudioside C contained in the stevia extract is 0.2 mass% or less.

11. The taste-improving method according to claim 9 or 10, wherein the taste to be improved is at least one selected from the group consisting of the bitterness, lingering sweetness, richness, and quickness of sweetness expression of the stevia extract, preferably all of them.

**EP 3 616 532 B1**

**Patentansprüche**

1. Eine Süßstoffzusammensetzung, umfassend einen Stevia-Extrakt, umfassend 90 Massen-% oder mehr, vorzugsweise 95 Massen-% oder mehr von Rebaudiosid A und Mogrosid V, wobei Rebaudiosid A und Mogrosid V in der Süßstoffzusammensetzung in einem Massenverhältnis von Rebaudiosid A zu Mogrosid V von 96:4 bis 98:2 gemischt sind.

2. Eine Süßstoffzusammensetzung gemäß Anspruch 1, wobei die Gesamtmenge an anderen Steviolglykosiden, die im Stevia-Extrakt enthalten sind, 1 Massen-% oder weniger beträgt und die Gesamtmenge an Steviosid und Rebaudiosid C, die im Stevia-Extrakt enthalten sind, 0,2 Massen-% oder weniger beträgt.

3. Eine Zusammensetzung zur oralen Verabreichung oder zur Verwendung in einer Mundhöhle (orale Zusammensetzung), umfassend die Süßstoffzusammensetzung gemäß Anspruch 1 oder 2, wobei das Massenverhältnis von Rebaudiosid A zu Mogrosid V in der oralen Zusammensetzung 96:4 bis 98:2 beträgt.

4. Die orale Zusammensetzung gemäß Anspruch 3, die eine Nahrungsmittel- oder Getränkezusammensetzung ist.

5. Die orale Zusammensetzung gemäß Anspruch 3 oder 4, die Rebaudiosid A und Mogrosid V in einer Gesamtmenge von 10 ppm bis 5000 ppm umfasst.

6. Ein Verfahren zum Verleihen von Süße an eine Zusammensetzung zur oralen Verabreichung oder zur Verwendung in einer Mundhöhle (orale Zusammensetzung), wobei ein Stevia-Extrakt, der 95 Massen-% oder mehr von Rebaudiosid A und Mogrosid V umfasst, in der oralen Zusammensetzung in einem Massenverhältnis von Rebaudiosid A zu Mogrosid V von 96:4 bis 98:2 gemischt sind.

7. Das Verfahren zum Verleihen von Süße gemäß Anspruch 6, wobei die Gesamtmenge anderer Steviolglykoside, die in dem Stevia-Extrakt enthalten sind, 1 Massen-% oder weniger beträgt und die Gesamtmenge an Steviosid und Rebaudiosid C, die in dem Stevia-Extrakt enthalten sind, 0,2 Massen-% oder weniger beträgt.

8. Das Verfahren zum Verleihen von Süße gemäß Anspruch 6 oder 7, wobei die orale Zusammensetzung Rebaudiosid A und Mogrosid V in einer Gesamtmenge von 10 ppm bis 5000 ppm umfasst.

9. Ein Verfahren zur Geschmacksverbesserung eines Stevia-Extrakts, umfassend 90 Massen-% oder mehr, vorzugsweise 95 Massen-% oder mehr von Rebaudiosid A, umfassend den Schritt:
   Mischen des Stevia-Extrakts mit Mogrosid V in einem Massenverhältnis von Rebaudiosid A zu Mogrosid V in einem Bereich von 96:4 bis 98:2.

10. Das Verfahren zur Geschmacksverbesserung gemäß Anspruch 9, wobei die Gesamtmenge anderer Steviolglykoside, die im Stevia-Extrakt enthalten sind, 1 Massen-% oder weniger beträgt und die Gesamtmenge an Steviosid und Rebaudiosid C, die im Stevia-Extrakt enthalten ist, 0,2 Massen-% oder weniger beträgt.

11. Das Verfahren zur Geschmacksverbesserung gemäß Anspruch 9 oder 10, wobei der zu verbessernde Geschmack mindestens einer ist, ausgewählt aus der Gruppe bestehend aus Bitterkeit, Nachsüße, Intensität und Schnelligkeit des Ausdrucks der Süße des Stevia-Extrakts, vorzugsweise alle davon.

**Revendications**

1. Composition d'édulcorant comprenant un extrait de stévia comprenant 90 % en masse ou plus, de préférence 95 % en masse ou plus de rébaudioside A et de mogroside V, dans laquelle le rébaudioside A et le mogroside V sont mélangés dans la composition d'édulcorant à un rapport en masse de rébaudioside A au mogroside V de 96:4 à 98:2.

2. Composition d'édulcorant selon la revendication 1, dans laquelle la quantité totale d'autres glycosides de stéviol contenus dans l'extrait de stévia est de 1 % en masse ou moins, et la quantité totale de stévioside et de rébaudioside C contenus dans l'extrait de stévia est de 0,2 % en masse ou moins.

3. Composition pour administration orale ou utilisée pour une cavité buccale (composition orale), comprenant la composition d'édulcorant selon la revendication 1 ou 2, dans laquelle le rapport de masse entre le rébaudioside A et le mogroside V dans la composition orale est compris entre 96:4 et 98:2.

4. Composition orale selon la revendication 3, qui est une composition alimentaire ou de boisson.

5. Composition orale selon la revendication 3 ou 4, qui comprend le rébaudioside A et le mogroside V dans une quantité totale de 10 ppm à 5000 ppm.

6. Procédé pour conférer un goût sucré à une composition destinée à être administrée par voie orale ou utilisée pour une cavité buccale (composition orale), dans lequel un extrait de stévia comprenant 95 % en masse ou plus de rébaudioside A et de mogroside V sont mélangés dans la composition orale à un rapport en masse de rébaudioside A au mogroside V de 96:4 à 98:2.

7. Procédé pour conférer un goût sucré selon la revendication 6, dans lequel la quantité totale d'autres glycosides de stéviol contenus dans l'extrait de stévia est inférieure ou égale à 1 % en masse, et la quantité totale de stévioside et de rébaudioside C contenus dans l'extrait de stévia est inférieure ou égale à 0,2 % en masse.

8. Procédé pour conférer un goût sucré selon la revendication 6 ou 7, dans lequel la composition orale comprend du rébaudioside A et du mogroside V dans une quantité totale de 10 ppm à 5000 ppm.

9. Procédé pour améliorer le goût d'un extrait de stévia comprenant 90 % en masse ou plus, de préférence 95 % en masse ou plus de rébaudioside A, comprenant l'étape consistant à :
mélanger l'extrait de stévia avec le mogroside V dans un rapport de masse entre le rébaudioside A et le mogroside V compris entre 96:4 et 98:2.

10. Procédé pour améliorer le goût selon la revendication 9, dans lequel la quantité totale d'autres glycosides de stéviol contenus dans l'extrait de stévia est de 1 % en masse ou moins, et la quantité totale de stévioside et de rébaudioside C contenus dans l'extrait de stévia est de 0,2 % en masse ou moins.

11. Procédé pour améliorer le goût selon la revendication 9 ou 10, dans lequel le goût à améliorer est au moins un élément choisi dans le groupe constitué de l'amertume, la douceur persistante, la richesse et la rapidité de l'expression du goût sucré de l'extrait de stévia, de préférence tous ces éléments.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10146165 A
- JP 2000287642 A
- JP 2001321115 A
- JP 2002223721 A
- JP 2006238828 A
- JP 2011024445 A
- WO 2009063921 A
- JP 2016041073 A
- JP 2011115142 A